# EUROPÄISCHE PATENTSCHRIFT

(11) **EP 3 380 005 B1**
(45) Veröffentlichungstag und Bekanntmachung des Hinweises auf die Patenterteilung: **30.08.2023**
(21) Anmeldenummer: 16810234.1
(22) Anmeldetag: 15.11.2016
(51) Int. Cl.: A61B 5/00, A61B 5/30, A61B 8/00, A61B 8/08, A61B 8/12, A61N 5/06, G16H 50/30

(54) **KONTROLLANORDNUNG UMFASSEND EINE SONDE MIT EINER LICHTQUELLE ODER EINER SCHALLQUELLE UND EINE SONDE ZUR ERFASSUNG EINES ANTWORTSIGNALS**
CONTROL ASSEMBLY COMPRISING A PROBE WITH A LIGHT SOURCE OR AN ACOUSTIC SOURCE AND A PROBE FOR DETECTING A RESPONSE SIGNAL
ENSEMBLE DE CONTRÔLE COMPRENANT UNE SOURCE DE LUMIÈRE OU UNE SOURCE ACOUSTIQUE ET UNE SONDE POUR DÉTECTER UN SIGNAL DE RÉPONSE

(30) Priorität: 25.11.2015 LU 92886
(43) Veröffentlichungstag der Anmeldung: 03.10.2018
(73) Patentinhaber: Universitätsmedizin der Johannes Gutenberg-Universität, 55131 Mainz (DE); Hochschule Trier, 54293 Trier (DE)
(72) Erfinder: KOCH, Klaus Peter, 66687 Wadern (DE); KNEIST, Werner, 55116 Mainz (DE)
(74) Vertreter: Müller, Jochen
(86) Internationale Anmeldenummer: PCT/DE2016/100534
(87) Internationale Veröffentlichungsnummer: WO 2017/088850

(56) Entgegenhaltungen:
- US-A- 5 810 731
- US-A1- 2013 197 321
- US-A1- 2015 289 929
- US-B1- 6 436 129

## Beschreibung

Die Erfindung bezieht sich auf eine Kontrollanordnung zur intraoperativen Stimulierbarkeit von durch das Nervensystem gesteuerten Funktionen mit mindestens einer Sonde zum Einbringen in den Körper und mindestens einem Sensor. Zum intraoperativen Monitoring der Nerven, insbesondere der autonomen Beckennerven, finden unterschiedliche Kontakt-Sonden zur elektrischen Stimulation Anwendung. Im Weiteren werden verschiedene Elektroden zur Ableitung von Muskelsignalen an Zielorganen oder mechanische Sensoren, beispielsweise Drucksensoren, zur Messwerterfassung am Zielorgan eingesetzt.

Aus der DE 10 2010 019 796 B4 ist eine Kontrollanordnung zur intraoperativen Stimulierbarkeit von durch das vegetative Nervensystem gesteuerten Funktionen im Beckenbereich bekannt. Die Kontrollanordnung umfasst mindestens jeweils eine Stimulationselektrode und Sensoren zur Messung von Signalen. Die mindestens eine Stimulationselektrode ist zum Einbringen in den Beckenraum während einer Operation ausgeführt und die zur intraoperativen Überwachung eingerichtete Kontrollanordnung umfasst zur EMG-Messung ausgebildete Sensoren, nämlich mindestens einen Sensor zur Messung der Blasenfunktion und mindestens einen Sensor zur Messung der Aktivität des Sphincter Ani.

Bei der EMG-Messung handelt es sich um eine elektrophysiologische Methode in der neurologischen Diagnostik, bei der die elektrische Muskelaktivität gemessen wird.

Die elektrische Stimulation von Nerven durch eingebrachte Sonden ist insofern nachteilig, als eine Verletzungsgefahr durch eine mechanische Irritation des Gewebes und die Gefahr einer Limitation der visuellen Selektivität durch die Größe der Sonde und der resultierenden elektrischen Felder im Vergleich zum Umfang der Nerven besteht. Im Weiteren entstehen bei der elektrischen Stimulation zum Monitoring von Nerven elektrische Stimulationsartefakte des Stimulationspulses bei der Signalaufzeichnung in der Zielregion sowie mechanische Artefakte durch die die Berührung der Sonde. Diese Artefakte erschweren die Analyse der aufgezeichneten Signale, zum Beispiel beim musculus sphincter ani bzw. musculus sphincter ani internus. Dies ist insbesondere bei der Analyse der direkten Antworten der Nervenstimulation problematisch, da kleine Signalanteile durch die abklingenden Stimulationsartefakte gestört werden. Diese Signalanteile können zwar unterdrückt werden, hierbei geht jedoch ein Teil des Nutzsignals verloren. Besonders kritisch ist dies jedoch bei der indirekten Änderung der Nervenaktivität (Neuromodulation), da hier die Stimulationsantworten zeitlich nicht mit den einzelnen Stimulationspulsen korrelieren. Darüber hinaus ist die Orientierung des Stroms, die insbesondere beim Einsatz bipolarer gabelförmiger Sonden dazu führt, dass das Stimulationsresultat von der Elektrodenausrichtung abhängig ist.

Bei der Detektion kommen Elektroden zur Ableitung der Muskelaktivität oder mechanische Sensoren zum Einsatz, die gegen mechanische Artefakte, beispielsweise durch eine atmungsbedingte oder durch das medizinische Personal verursachte Bewegung des Patienten, empfindlich sind.

Die US 6 436 129 B1 offenbart ein Verfahren zur Stimulierung des Nervenwachstums, bei dem ein Gewebebereich neben einem Bereich geschädigter Nerven mit Wärme stimulieren wird, um ein Wachstum der unbeschädigten Nerven in den geschädigten Gewebebereich anzuregen. Zum Einbringen von Wärme werden beispielsweise Heizelektroden, RF-Elektroden, Infrarot-Sonden, Mikrowellensonden, Ultraschallsender und optische Fasersonden verwendet. Um eine Überhitzung des stimulierten Gewebes zu verhindern, wird eine Elektrolytlösung bzw. ein Kühlfluid eingesetzt oder es wird ein Steuermechanismus zur Steuerung der auf den behandelten Nerv einwirkenden Temperatur verwendet.

Im Weiteren zeigt die US 2015/289 929 A1 Sonden und Verfahren, mit denen die Empfindlichkeit eines Organs in einem Körper auf einen Reiz überwacht bzw. Zielgewebe verändert wird, beispielsweise um einen Krebstumor zu behandeln. Eine oder mehrere Sonden können eine elektrophysiologische Aktivität bei einem Körper überwachen. Das System umfasst einen mit mehreren Sonden gekoppelten Prozessor, um Signale von den Standorten der Sonden zu empfangen und die Position einer elektrophysiologisch reichen Region, die zu einer oder mehreren Sonden benachbart ist, zu bestimmen. Die Sonden können faseroptische Elemente umfassen, die mit einer Lichtquelle und/oder einem Laser gekoppelt sind, um eine optische Strahlung zu dem Zielgewebe zu dessen Überwachung oder Behandlung zu führen. Im Weiteren kann eine Sonde zur Überwachung der lokalen elektrophysiologischen Aktivität, eine oder mehrere Mikroelektroden und/oder eine anregende und/oder abtragende Elektrode zur Stimulation und/oder Behandlung einer lokalen Gewebestelle umfassen.

Es besteht darüber hinaus die Möglichkeit sensorische Schaltungen, beispielsweise während eines chirurgischen Eingriffs oder eines Überwachungsverfahren, zu konfigurieren, die beispielsweise Strom, Spannung, Impedanz oder Temperatur zwischen einem oder mehreren Bereichen einer Sonde oder zwischen zwei oder mehreren Sonden oder zwischen einer Sonde und einer externen Elektrode (beispielsweise einer extern angeordneten Referenzelektrode) überwachen.

Aus der US 2013/197 321 A1 sind Verfahren und Systeme zur Überwachung und/oder Behandlung von Störungen der oberen Atemwege, wie Apnoe, Dysphagie, Reflux und/oder Schnarchen, bekannt. Eine Behandlung erfolgt durch die Stimulation von Muskeln und Sinnesrezeptoren zur Auslösung von Reflexen, beispielsweise eines Schluckreflexes. Geeignete neurale Signalmessvorrichtungen umfassen elektrische Sensoren, wie beispielsweise Nervenmanschetten, optische Sensoren, beispielsweise Lichtwellenleiter, die in Kombination mit spannungs- oder stromempfindlichen Farbstoffen, die in die Nerven injiziert werden, verwendet werden, biologische Sensoren, und mechanische Sensoren. Im Weiteren können Aufzeichnungselektroden zur Überwachung von Signalquellen verwendet werden.

Der Erfindung liegt die Aufgabe zugrunde, eine Kontrollanordnung zum Monitoring von Nerven der eingangs genannten Art zu schaffen, die die vorgenannten Nachteile vermeidet und dadurch einen ungestörten Operationsablauf mit möglichst wenigen Unterbrechungen Erfindungsgemäß wird die Aufgabe bei einer Kontrollanordnung mit den Merkmalen des Anspruchs 1 gelöst.

Eine Kontrollanordnung, die zur intraoperativen Stimulierbarkeit von durch das Nervensystem gesteuerten Funktionen geeignet ist, umfasst mindestens einer in einen Körper einbringbaren Sonde, wobei
- eine erste Sonde mit mindestens einer mit einer mit einer Auswerteeinrichtung gekoppelten Steuereinrichtung verbundenen ersten Lichtquelle oder einer Schallquelle gekoppelt ist, wobei mit der Lichtquelle oder der Schallquelle ein Stimulationssignal in Form von zur Unterscheidung von neuronalem Gewebe von nicht neuronalem Gewebe geeigneten Strahlungen erzeugbar ist, wobei die der ersten Sonde zugeordnete Steuer- und Auswerteeinrichtung mit einem ersten Sensor zur Erfassung einer reflektierten Lichtstrahlung durch eine optische Faser oder mit Schallerfassungsmitteln zur Erfassung reflektierter Schallsignale verbunden ist, wobei der erste Sensor mit der zugeordneten Auswerteeinrichtung eine Temperaturmes- und Temperaturüberwachungsfunktion umfasst, die regelnd auf die Steuereinrichtung und damit auf die emittierte Strahlung einwirkt oder der erste Sensor Mittel zum Empfang reflektierter Schallsignale umfasst, und
- eine zweite Sonde zur Erfassung eines Antwortsignals eines Zielorgans ausgebildet ist, wobei die zweite Sonde mindestens eine EMG-Ableitelektrode umfasst oder zur Erfassung eines Drucksignals ausgebildet ist oder mindestens eine mit einer zweiten Lichtquelle verbundene optische Faser umfasst, wobei die zweite Lichtquelle zur Abgabe einer Lichtstrahlung im infraroten Wellenlängenbereich an eine Steuereinrichtung angeschlossen ist, die wiederum mit einer mit einem zweiten Sensor verbundenen Auswerteeinrichtung gekoppelt ist,
- wobei die beiden Sonden über ihre zugeordnete Steuer- und Auswerteeinrichtung mit einem Überwachungsrechner der Kontrollanordnung in Verbindung stehen.

Die Unteransprüche stellen vorteilhafte Ausgestaltungen der Erfindung dar.

In Ausgestaltung ist der ersten Sonde eine Steuer- und Auswerteeinrichtung zugeordnet, die mit einem ersten Sensor zur Erfassung einer reflektierten Lichtstrahlung durch eine optische Faser oder mit Schallerfassungsmitteln zur Erfassung reflektierter Schallsignale verbunden ist.

Durch die optische oder hochfrequente Stimulation oder die Ultraschallstimulation des Gewebes, die die aus dem Stand der Technik bekannte elektrische Stimulation ersetzt, ist die Entstehung von Artefakten reduziert. Die zum Einsatz kommende optische Strahlung im sichtbaren oder nicht-sichtbaren Wellenlängenbereich bzw. die verwendeten Schallwellen weisen keine Orientierung auf, weshalb der zu stimulierende Bereich nicht von der Ausrichtung der Sonde zu dem zu stimulierenden Gewebe, insbesondere dem neuronalen Gewebe, abhängt, sondern lediglich durch den Abstand der Sonde zu dem Nerv sowie den Stimulationsparametern bestimmt ist. Da im Gegensatz zu den aus dem Stand der Technik bekannten Stimulationsmethoden beim Einsatz der optischen Strahlung bzw. der verwendeten Schallwellen kein direkter Kontakt zu dem zu stimulierenden Gewebe erforderlich ist, ist die Stimulation für den Patienten schonender, wobei es sich bei dem Patienten selbstverständlich sowohl um einen Menschen als auch ein Tier handeln kann.

Die zweite Sonde ist beispielsweise zur Messung der Aktivität des Zielorgans, insbesondere dem m. sphincter ani bzw. m. sphincter ani internus, beispielsweise in Form mindestens einer EMG-Ableitelektrode oder zur Erfassung eines Drucksignals ausgebildet. Bei der EMG-Ableitelektrode kann es sich um eine bipolare Elektrode handeln, die einen ersten Pol und einen zu dem Außenumfang des ersten Pols konzentrisch sowie elektrisch isoliert angeordneten einen zweiten Pol aufweist. Die bipolare Ableitelektrode eignet sich zur Platzierung in glatter Muskulatur. wie beispielsweise dem m. sphincter ani internus, etwa durch Eintreiben. Alternativ können auch zwei einpolige Elektroden verwendet werden, oder Elektroden mit mehr als zwei Polen. Die bipolare Ableitelektrode kann beispielsweise einen Durchmesser von ungefähr 0,1 bis ungefähr 1 mm haben, z. B. ungefähr 0,5 mm, und eine Länge von ungefähr 5 bis ungefähr 30 mm, z.B. ungefähr 15 bis ungefähr 25 mm. Ein beispielsweise auf dem Piezoeffekt basierender Drucksensor ist insbesondere für eine Messung des Drucks in der Blase geeignet ist, wobei ein Druckanstieg nach einer Nervenstimulation anzeigt, dass die Nervenbahnen zum Detrusor vesicae noch funktionieren. Hierzu kann vorzugsweise ein über einen Katheterschlauch mit dem Drucksensor verbundener Blasenkatheter vorgesehen sein.

Zur Erfassung der Stimulationsantwort ist die Sonde beispielsweise licht-oder schallempfangend ausgebildet, wobei an dem Zielorgan auch eine Ableitung elektrischer bzw. elektromagnetischer Signale mittels der Sonde erfolgen kann, die mit einem entsprechenden Leiter zum Empfangen der Signale ausgestattet ist. Es ist auch möglich, die Stimulationsantwort über mechanische Erfassungsmittel der zweiten Sonde abzuleiten. Zweckmä-ßigeniveise wird über eine optische Faser der zweiten Sonde eine Überwachung des Zielorgans vorgenommen und mittels optischer Messungen physiologische Änderungen über Differenzen im Reflektions- oder Absorptionsspektrum vorgenommen, insbesondere Informationen über Änderungen des Muskeltonus, z. B. der Durchblutung, empfangen. Die optischen Signale können sich im sichtbaren oder nicht-sichtbaren Wellenlängenbereich bewegen.

Der Überwachungsrechner dient zur Erfassung, Auswertung und Dokumentation der Monitoring-Daten.

Mittels dem der Sonde zugeordneten, licht- oder schallempfangend ausgebildeten Sensors sind Reflexionsmessungen durchführbar Der Sensor ist mit der Steuer- und Auswerteeinrichtung verbunden, um beispielsweise eine Schädigung des stimulierten Gewebes zu vermeiden. Mittels des Sensors lässt sich beispielsweise ein Temperatur-Monitoring vornehmen und anhand der erfassten Daten kann auch das Stimulationssignal angepasst werden.

Wird in die Sonde eine Lichtstrahlung im sichtbaren Wellenlängenlängenbereich eingekoppelt, besteht die Möglichkeit, durch diesen Hilfslichtstrahl, der selbstverständlich neben der Stimulationsstrahlung vorliegen kann, die Stimulationsstelle zu visualisieren.

Um die Stimulationsparameter zu ändern, ist bevorzugt die Steuer- und Auswerteeinrichtung zur Modulation des Stimulationssignals ausgebildet. Durch die Modulation kann in Form von Amplituden, eine Frequenz, eine Pulsbreite oder eine Pulsform des Stimulationssignals, also der optischen Strahlung, der hochfrequenten Schallwellen bzw. der Ultraschallwellen erfolgen, wobei die Stärke des Stimulationssignals verändert wird, bis hin zum Ein- und Ausschalten. Durch die Modulation des Stimulationssignals lassen sich die von dem mindestens einen Sensor empfangenen Stimulationsantworten relativ sicher von Artefakten unterscheiden. Mittels der Modulation des Stimulationssignals lässt sich auch zwischen einer Nervenstimulation und einer Nervenblockade durch ein hochfrequentes Signal auswählen.

Aufgrund der reflektierten Signale kann mittels der Steuer- und Auswerteeinrichtung bereits vor der Stimulation des Gewebes eine Unterscheidung zwischen neuronalem und nicht neuronalem Gewebe getroffen werden. Zur Auswertung werden unterschwellige Signale genutzt, die keine Stimulation der Nerven auslösen, wodurch eine effektive intraoperative Stimulation mit einem gesicherten Operationsfluss durch die Vermeidung unnötiger Unterbrechungen möglich ist.

Aufgrund der Temperaturmessung und Temperaturüberwachung ist die Gefahr einer temperaturbedingten Schädigung des stimulierten Gewebes aufgrund der eingesetzten Strahlung wesentlich reduziert. Zur Temperaturermittlung kann beispielsweise ein mit der Steuer- und Auswerteeinrichtung verbundener Infrarotdetektor verwendet werden, wobei eine Aufzeichnung der ermittelten Daten erfolgt. Selbstverständlich lässt sich das gesamte Monitoring mittels der Steuer- und Auswerteeinrichtung vornehmen, die auch mit Speicher-, Eingabe- und Ausgabeeinrichtungen gekoppelt sein kann.

Das Stimulationssignal und das resultierenden Antwortsignal des Zielorgans ab Ableitort müssen nicht zwingend in einer identischen Art und Weise zusammenhängen, sondern zueinander korrelieren. Dementsprechend moduliert der Fachmann geeignete Signale, die auch eine Auswertung der Antworten mit möglichst geringen Störungen ermöglichen.

Dementsprechend umfasst die Kontrollanordnung im Wesentlichen eine erste Sonde zur Stimulation von Gewebe und einen ersten Sensor zur Durchführung von Reflexionsmessungen sowie eine zweite Sonde mit einem zweiten Sensor zur Überwachung des Muskeltonus eines Zielorgans, wobei die beiden Sonden zum Einbringen in den Körper geeignet, insbesondere zum Monitoring mit Steuer- und Auswerteeinrichtungen sowie einem Überwachungsrechner gekoppelt und mit unterschiedlichen Parametern betreibbar sind.

Die zuvor und nachstehend in einem Ausführungsbeispiel beschriebene sowie beanspruchte Kontrollanordnung ist als solche benannt und kann selbstverständlich auch als eine Kontrolleinrichtung bezeichnet werden ohne den Rahmen der Erfindung zu verlassen. Die Kontrollanordnung oder Kontrolleinrichtung umfasst die eindeutig zur Erfindung gehörende Steuer- und Auswerteeinrichtung, den Überwachungsrechner und die erste und die zweite Sonde mit ihren zugeordneten Sensoren. Die Eignung der Sonden zum Einbringen in den Körper, beispielsweise durch eine minimalinvasive Öffnung, soll insbesondere einen Rückschluss auf deren äußere Beschaffenheit, insbesondere deren Geometrie sowie die Auswahl des Materials und deren Möglichkeit zur Reinigung beschreiben.

Insbesondere wird kein therapeutisches Verfahren zur Behandlung eines menschlichen oder tierischen Körpers beansprucht. Die anhand eines Gewebes beschriebene Wirkung oder Funktion einer Strahlung soll lediglich die physikalischen Parameter der Strahlung funktional beschreiben.

Es versteht sich, dass die vorstehend genannten und nachstehend noch zu erläuternden Merkmale nicht nur in der jeweils angegebenen Kombination, sondern auch in anderen Kombinationen verwendbar sind. Der Rahmen der Erfindung ist nur durch die Ansprüche definiert.

Die Erfindung wird im Folgenden anhand eines Ausführungsbeispiels unter Bezugnahme auf die zugehörige Zeichnung näher erläutert.

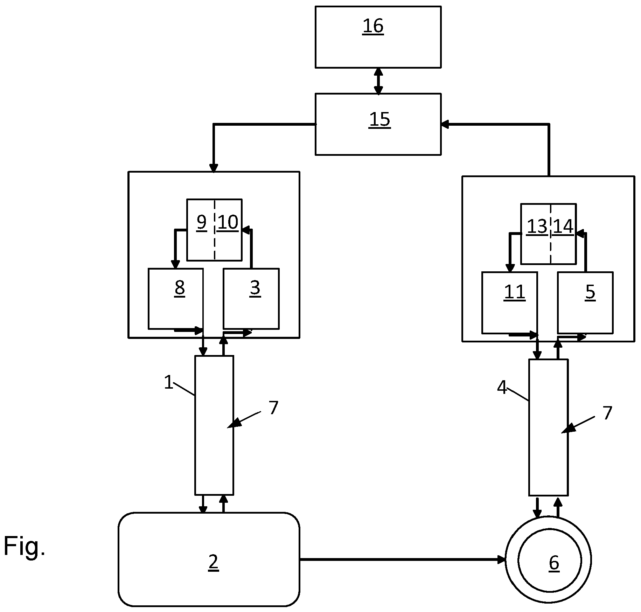

Die einzige Fig. der Zeichnung zeigt eine schematische Darstellung einer Kontrollanordnung nach der Erfindung.

Die Kontrollanordnung umfasst eine erste Sonde 1 zur Stimulation von Gewebe 2, vorzugsweise von neuronalem Gewebe 2, und einen ersten Sensor 3 zur Durchführung von Reflexionsmessungen sowie eine zweite Sonde 4 mit einem zweiten Sensor 5 zur Überwachung des Muskeltonus eines Zielorgans 6, wobei die beiden Sonden 1 zum Einbringen in den Körper geeignet sind, insbesondere bei einer minimal-invasiven Operation, und bei Bedarf auch in ein gemeinsames Gehäuse zu integrieren sind.

Die erste Sonde 1 umfasst mindestens eine optische Faser 7, die mit einer Lichtquelle 8 in Verbindung steht, um eine Lichtstrahlung zu emittieren. Die Lichtquelle 8 ist zur Abgabe einer Lichtstrahlung im infraroten oder sichtbaren Wellenlängenbereich an eine Steuereinrichtung 9 angeschlossen, die wiederum mit einer Auswerteeinrichtung 10 gekoppelt ist, wobei selbstverständlich die Steuereinrichtung 9 und die Auswerteeinrichtung 10 eine Baueinheit darstellen können.

Mittels der ersten Sonde 1 bzw. der mit der Steuereinrichtung 9 verbundenen Lichtquelle 8 werden Strahlungen moduliert, die zur Unterscheidung von neuronalem Gewebe von nicht neuronalem Gewebe geeignet sind, eine Stimulation von neuronalem Gewebe ermöglichen oder eine Ausleuchtung mittels Lichtstrahlung im sichtbaren Wellenlängenlängenbereich sicherstellen. Die reflektierte Lichtstrahlung wird durch die mindestens eine optische Faser 7 zu dem mit der Auswerteeinrichtung 10 verbundenen ersten Sensor 3 geleitet. Um eine temperaturbedingte Schädigung des Gewebes 2 zu vermeiden, umfasst der erste Sensor 3 mit der zugeordneten Auswerteeinrichtung 10 eine Temperaturmess- und Temperaturüberwachungsfunktion, die regelnd auf die Steuereinrichtung 9 und damit auf die emittierte Strahlung einwirkt.

Für den Fachmann ist es ersichtlich, dass die erste Sonde 1 mit einer Schallwellenübertragungsvorrichtung mit einem zugeordneten Schallgenerator, der an die Steuereinrichtung 9 angeschlossen ist, ausgestattet werden kann, um eine Unterscheidung von neuronalem Gewebe und nicht neuronalem Gewebe sowie eine Stimulation von neuronalem Gewebe durchzuführen. Hierbei umfasst der erste Sensor 3 selbstverständlich Mittel zum Empfang reflektierter Schallsignale und ist mit der Auswerteeinrichtung 10 verbunden. Vorzugsweise sind der ersten Sonde auch bei der Nutzung von Schallwellen im hochfrequenten Bereich bzw. im Ultraschallbereich, optische Fasern 7 und eine Lichtquelle 8 zugeordnet, um zum einen eine Beleuchtung und zum anderen eine Temperaturmessung des bestrahlten Gewebes 2 zu realisieren.

Die zweite Sonde 4 weist grundsätzlich einen sehr ähnlichen Aufbau wie die erste Sonde 1 auf und umfasst ebenfalls mindestens eine mit einer Lichtquelle 11 verbundene optische Faser 12, wobei die Lichtquelle 11 zur Abgabe einer Lichtstrahlung im infraroten Wellenlängenbereich an eine Steuereinrichtung 13 angeschlossen, die wiederum mit einer mit dem zweiten Sensor 4 verbundenen Auswerteeinrichtung 14 gekoppelt ist. Mittels dieser zweiten Sonde 4 wird der durch die Stimulation des neuronalen Gewebes mit der ersten Sonde 1 hervorgerufene Muskeltonus des Zielorgans 6 überwacht, wozu eine amplitudenmodulierte Lichtstrahlung emittiert wird, um beispielsweise die Durchblutung des Zielorgans 6 mittels einer Spektralanalyse zu überwachen.

Die erste Sonde 1 mit dem ersten Sensor 3, der Steuereinrichtung 9 sowie der Auswerteeinrichtung 10 bilden eine das Gewebe 2 betreffende Stimulations- und Kontrolleinheit und die zweite Sonde 4 mit dem zweiten Sensor 5, der zugeordneten Steuereinrichtung 9 sowie der gekoppelten Auswerteeinrichtung 14 bilden eine Überwachungseinheit für den Muskeltonus. Die beiden Steuereinrichtungen 9, 13 sowie die beiden Auswerteeinrichtungen 10, 14 können in ein Gerät integriert sein, das einen Überwachungsrechner 15 mit zugeordneten Eingabe- und Ausgabevorrichtungen 16 und/oder Schnittstellen umfasst oder an einen solchen Überwachungsrechner 15 angeschlossen sein, beispielsweise über ein Netzwerk.

### Bezugszeichen

- 1.: erste Sonde
- 2.: Gewebe
- 3.: erster Sensor
- 4.: zweite Sonde
- 5.: zweiter Sensor
- 6.: Zielorgan
- 7.: Faser
- 8.: Lichtquelle
- 9.: Steuereinrichtung
- 10.: Auswerteeinrichtung
- 11.: Lichtquelle
- 12.: Faser
- 13.: Steuereinrichtung
- 14.: Auswerteeinrichtung
- 15.: Überwachungsrechner
- 16.: Eingabe- und Ausgabevorrichtungen

## Patentansprüche

1. Kontrollanordnung geeignet zur intraoperativen Stimulierbarkeit von durch das Nervensystem gesteuerten Funktionen mit mindestens einer in einen Körper einbringbaren Sonde (1, 4), wobei
- die erste Sonde (1) mit mindestens einer mit einer Auswerteeinrichtung (10) gekoppelten Steuereinrichtung (9) verbundenen ersten Lichtquelle (8) oder einer Schallquelle gekoppelt ist, wobei mit der Lichtquelle (8) oder der Schallquelle ein Stimulationssignal in Form von zur Unterscheidung von neuronalem Gewebe von nicht neuronalem Gewebe geeigneten Strahlungen erzeugbar ist, wobei die der ersten Sonde (1) zugeordnete Steuer- und Auswerteeinrichtung (9,10) mit einem ersten Sensor (3) zur Erfassung einer reflektierten Lichtstrahlung durch eine optische Faser (7) oder mit Schallerfassungsmitteln zur Erfassung reflektierter Schallsignale verbunden ist, wobei die Steuer-und Auswerteeinrichtung (9, 10) dazu ausgebildet ist, aufgrund der von dem ersten Sensor (3) erfassten reflektierten Signale eine Unterscheidung von neuronalem und nicht neuronalem Gewebe zu treffen, wobei der erste Sensor (3) mit der zugeordneten Auswerteeinrichtung (10) eine Temperaturmess- und Temperaturüberwachungsfunktion umfasst, die regelnd auf die Steuereinrichtung (9) und damit auf die emittierte Strahlung einwirkt, und
- eine zweite Sonde (4) zur Erfassung einer Stimulationsantwort eines Zielorgans ausgebildet ist, wobei die zweite Sonde (4) mindestens eine EMG-Ableitelektrode umfasst oder zur Erfassung eines Drucksignals ausgebildet ist oder mindestens eine mit einer zweiten Lichtquelle (11) verbundene optische Faser (12) umfasst, wobei die zweite Lichtquelle (11) zur Abgabe einer Lichtstrahlung im infraroten Wellenlängenbereich an eine Steuereinrichtung (13) angeschlossen ist, die wiederum mit einer mit einem zweiten Sensor (5) verbundenen Auswerteeinrichtung (14) gekoppelt ist,
- wobei die beiden Sonden (3, 4) über ihre zugeordnete Steuer-(9, 13) und Auswerteeinrichtung (10, 14) mit einem Überwachungsrechner (15) der Kontrollanordnung in Verbindung stehen.

2. Kontrollanordnung nach Anspruch 1, wobei die Steuer- (9, 13) und Auswerteeinrichtung (10, 14) zur Modulation des Stimulationssignals ausgebildet ist wobei insbesondere das Signal der Lichtquelle (8, 11) oder der Schallquelle frequenz- oder pulsbreiten oder pulsform- oder amplituden-modulierbar ist.

3. Kontrollanordnung nach einem der Ansprüche 1 oder 2, wobei die Sonde (1, 4) durch eine minimalinvasive Öffnung einführbar ist.

4. Kontrollanordnung nach einem der Ansprüche 1 bis 3, wobei die erste und die zweite Sonde (1, 4) mit unterschiedlichen Parametern betreibbar sind.

5. Kontrollanordnung nach einem der Ansprüche 1 bis 4, wobei eine Lichtstrahlung im infraroten und/oder im sichtbaren Wellenlängenbereich in die Sonde (1 ,4) einkoppelbar ist.

## Claims

1. Monitoring assembly which is suitable for being able to intraoperatively stimulate functions controlled by the nervous system and has at least one probe (1, 4) which can be inserted into a body, wherein
- the first probe (1) is coupled to at least a first light source (8), which is connected to control apparatus (9) coupled to an evaluation apparatus (10), or to a sound source, wherein a stimulation signal in the form of radiation suitable for distinguishing neuronal tissue from non-neuronal tissue can be generated using the light source (8) or the sound source, wherein the control and evaluation apparatus (9, 10) associated with the first probe (1) is connected to a first sensor (3) for detecting reflected light radiation by means of an optical fiber (7) or to sound detection means for detecting reflected sound signals, wherein the control and evaluation apparatus (9, 10) is designed to distinguish between neuronal and non-neuronal tissue on the basis of the reflected signals detected by the first sensor (3), wherein the first sensor (3) having the associated evaluation apparatus (10) comprises a temperature measurement and temperature monitoring function which acts in a regulating manner on the control apparatus (9) and thus on the emitted radiation, and
- a second probe (4) is designed to detect a stimulation response of a target organ, wherein the second probe (4) comprises at least one EMG derivation electrode or is designed to detect a pressure signal or comprises at least one optical fiber (12) which is connected to a second light source (11), wherein the second light source (11) for emitting light radiation in the infrared wavelength range is connected to a control apparatus (13) which is in turn coupled to an evaluation apparatus (14) connected to a second sensor (5),
- wherein the two probes (3, 4) are connected to a monitoring computer (15) of the monitoring assembly via their associated control apparatus (9, 13) and evaluation apparatus (10, 14).

2. Monitoring assembly according to claim 1, wherein the control apparatus (9, 13) and evaluation apparatus (10, 14) are designed to modulate the stimulation signal, in particular wherein the signal of the light source (8, 11) or the sound source can be modulated in terms of frequency or pulse width or pulse shape or amplitude.

3. Monitoring assembly according to either claim 1 or claim 2, wherein the probe (1, 4) can be inserted through a minimally invasive opening.

4. Monitoring assembly according to any of claims 1 to 3, wherein the first and second probe (1, 4) can be operated using different parameters.

5. Monitoring assembly according to any of claims 1 to 4, wherein light radiation in the infrared and/or in the visible wavelength range can be coupled into the probe (1, 4).

## Revendications

1. Agencement de contrôle adapté pour la stimulation peropératoire de fonctions commandées par le système nerveux, comportant au moins une sonde (1, 4) pouvant être introduite dans un corps,
- la première sonde (1) étant couplée à au moins une première source lumineuse (8) ou une source sonore reliée à un dispositif de commande (9) couplé à un dispositif d'évaluation (10), un signal de stimulation sous forme de rayonnements adaptés pour différencier des tissus neuronaux de tissus non neuronaux pouvant être généré par la source lumineuse (8) ou la source sonore, le dispositif de commande et d'évaluation (9, 10) associé à la première sonde (1) étant relié à un premier capteur (3) pour la détection d'un rayonnement lumineux réfléchi par une fibre optique (7) ou à des moyens de détection sonore pour la détection de signaux sonores réfléchis, le dispositif de commande et d'évaluation (9, 10) étant configuré pour établir une distinction entre des tissus neuronaux et des tissus non neuronaux sur la base des signaux réfléchis détectés par le premier capteur (3), le premier capteur (3) avec le dispositif d'évaluation (10) associé comprenant une fonction de mesure de la température et de surveillance de la température qui agit de manière régulatrice sur le dispositif de commande (9) et donc sur le rayonnement émis, et
- une seconde sonde (4) étant configurée pour la détection d'une réponse de stimulation d'un organe cible, la seconde sonde (4) comprenant au moins une électrode de dérivation EMG ou étant configurée pour la détection d'un signal de pression ou comprenant au moins une fibre optique (12) reliée à une seconde source lumineuse (11), la seconde source lumineuse (11) étant connectée à un dispositif de commande (13) pour émettre un rayonnement lumineux dans la plage de longueurs d'onde infrarouge, lequel dispositif de commande est à son tour couplé à un dispositif d'évaluation (14) relié à un second capteur (5),
- les deux sondes (3, 4) étant en liaison avec un ordinateur de surveillance (15) de l'agencement de contrôle par l'intermédiaire de leur dispositif de commande (9, 13) et d'évaluation (10, 14) associé.

2. Agencement de contrôle selon la revendication 1, le dispositif de commande (9, 13) et d'évaluation (10, 14) étant configuré pour moduler le signal de stimulation, le signal de la source lumineuse (8, 11) ou de la source sonore pouvant en particulier être modulé en fréquence ou en largeur d'impulsion ou en forme d'impulsion ou en amplitude.

3. Agencement de contrôle selon l'une des revendications 1 ou 2, la sonde (1, 4) pouvant être introduite par une ouverture peu invasive.

4. Agencement de contrôle selon l'une des revendications 1 à 3, les première et seconde sondes (1,4) pouvant fonctionner avec des paramètres différents.

5. Agencement de contrôle selon l'une des revendications 1 à 4, un rayonnement lumineux dans la plage de longueurs d'onde infrarouge et/ou visible pouvant être couplé dans la sonde (1, 4).
